# FASCICULE DE BREVET EUROPEEN

(11) **EP 0 920 445 B1**
(45) Date de publication et mention de la délivrance du brevet: **02.01.2003**
(21) Numéro de dépôt: 96931091.1
(22) Date de dépôt: 23.08.1996
(51) Int. Cl.: C07K 5/065, A61K 7/48, A61K 38/05

(54) **PEPTIDES SYNTHETIQUES ET LEUR UTILISATION DANS LES COMPOSITIONS COSMETIQUES OU DERMOPHARMACEUTIQUES**
SYNTHETISCHE PEPTIDE UND IHRE VERWENDUNG IN KOSMETISCHEN ODER DERMOPHARMAZEUTISCHEN ZUSAMMENSETZUNGEN
SYNTHETIC PEPTIDES AND THEIR USE IN COSMETIC OR DERMOPHARMACEUTICAL COMPOSITIONS

(43) Date de publication de la demande: 09.06.1999
(73) Titulaire: SEDERMA S.A., 78610 Le Perray-en-Yvelines Cédex (FR)
(72) Inventeur: GREFF, Daniel, F-78490 Mere (FR)
(74) Mandataire: Laget, Jean-Loup
(86) Numéro de dépôt international: FR9601322
(87) Numéro de publication internationale: WO98007744

(56) Documents cités:
- WO-A-92/19254
- WO-A-94/09750
- FR-A- 2 668 365
- P. Clapes et al. 'Enzymatic Synthesis of Z-Kyothorphin Amide ' in: Peptides 1988 Proceedings of the 20th European Symposium September 4-9, 1988 Eds. G. Jung and E. Bayer XP002030026
- Sigma Chemie 1995 - Catalogus Biochemicalien, Organische Verbindingen voor Research en Diagnostica XP002030031
- DATABASE WPI Section Ch, Week 9133 Derwent Publications Ltd., London, GB; Class B05, AN 91-240366 XP002030027 & ES 2 020 148 A (CONSEJO SUPERIOR INVESTIGACION) , 16 Juillet 1991
- DATABASE WPI Section Ch, Week 9332 Derwent Publications Ltd., London, GB; Class B04, AN 93-252653 XP002030028 & JP 05 170 637 A (NARISU KESHOHIN KK) , 9 Juillet 1993

## Description

La peau est l'organe le plus grand du corps, mais aussi le plus exposé aux agressions diverses: irritations dues à l'environnement (pollution, allergies), aux intempéries (vent, pluie, froid, rayonnement solaire, dessèchement), aux traitements physiques (rasage, épilation, frottements, chocs). Tout en étant un organe de protection de l'organisme entier contre la déshydratation, contre l'invasion bactérienne ou moléculaire, la peau sert aussi à communiquer avec l'environnement et à enregistrer les sensations du toucher. Elle est donc bien innervée, les sensations de douleur ou d'inconfort sont bien localisées dans l'épiderme du corps entier.

Les analyses anatomiques, histologiques et physiologiques ont mis en évidence des cellules connectées au système nerveux qui pourraient être les cellules responsables, au moins partiellement, de la transmission des signaux du toucher, et surtout des douleurs. Ces cellules sont appelées "cellules de Merkel".

Des études récentes ont mis en évidence par immunoréactivité la présence d'un certain nombre de neuropeptides au niveau de l'épiderme, à proximité des cellules de Merkel. Parmi ces neuropeptides on trouve l'enképhaline, un peptide de séquence Tyr-Gly-Gly-Phe-Leu, dont l'activité reconnue à ce jour est la suppression des douleurs (effet analgésique) par action au niveau du récepteur de la morphine dans le cerveau. Ce peptide a été trouvé, en dehors du tissu cérébral, en d'autres endroits du corps, à la périphérie, et donc aussi dans la peau. Son rôle n'y est pas encore connu.

D'autres études ont montré que la libération du peptide au niveau du cerveau - lors d'un besoin inhérent à un effet analgésique - se fait par l'intermédiaire d'un autre peptide de séquence Tyr-Arg. Ce peptide ne se lie pas au récepteur morphinique lui-même, mais il induit la synthèse ou la libération de l'enképhaline et provoque ainsi la suppression rapide - mais éphémère - de la douleur. Ce peptide n'a été jusqu ' ici trouvé qu'au niveau du cerveau, et son activité n'a été décrite que par administration intracérébrale directe.

L'objet du présent brevet est la découverte que ce peptide, et surtout des dérivés lipophiles du peptide, possèdent une activité calmante, antalgique, quand ils sont appliqués sur la peau humaine dans une préparation cosmétique ou dermopharmaceutique adéquate.

Les peptides correspondent à la formule générale : R1-L-Tyr-L-Arg-R2 où R1= un groupe R3-C=O avec R3 = une chaîne alkyle de C1 à C20, linéaire ou ramifiée, saturée ou insaturée, hydroxylée ou non, ou avec R3 = un groupe aryle, aryle-alkyle ou alkyloxy, et où R2 = un groupe O-R4 avec R4= une chaîne alkyl de C1 à C20, ou R2 = un groupe NH2, NHX ou NXX avec X = une chaîne alkyle de C1 à C4.

Le peptide H-L-Tyr-L-Arg-OH non lipophile ne possède qu'une faible activité par application topique, puisqu'il ne pénètre que difficilement à travers la couche cornée. La modification qui consiste à attacher par liaison amide une chaîne grasse (alkyle) ou un résidu aromatique (aryle) ou leurs variantes alkyloxy ou aryloxy ou arylalkyloxy sur le NH de la tyrosine, et/ou d'attacher par liaison ester un alcool gras (alkyle) ou par liaison amide un groupe NH2 ou NHX ou NXX où X = une chaîne alkyle de C1 à C4, confère au peptide une affinité nettement supérieure pour l'épiderme et un pouvoir accru de pénétration qui augmente très fortement l'activité biologique du peptide.

Le peptide peut être obtenu par les voies de synthèse peptidique classiques ou par voie enzymatique.

A titre d'exemple, on peut estérifier l'arginine avec un alcool à chaîne courte (butyle) par condensation acide, puis on attache la Boc-tyrosine à l'ester d'arginine par la méthode classique d'ester activé (chloroformiate d'éthyle ou DCC), ensuite on attache l'acide palmitique par la méthode de chlorure d'acide pour obtenir le N-palmitoyl-L-Tyr-L-Arg- O-butyle. La synthèse par voie enzymatique utilise des enzymes de type chymotrypsine ou trypsine dans un milieu préférentiellement anhydre pour coupler les acides aminés Tyrosine et Arginine, puis on procède à l'acylation de la tyrosine pour obtenir le N-acyl-L-Tyr-L-Arg.

L'homme de l'art de la synthèse peptidique sait préparer les dérivés de la formule générale indiquée par les méthodes connues.

Le peptide et ses dérivés, objets du présent brevet doivent être véhiculés par une préparation cosmétique ou dermopharmaceutique adéquate. Selon la nature des résidus R1 et R2 et X il possède des solubilités variables. La réalisation préférentielle de l'invention est constituée par les peptides de nature N-acyl^{A}-L-Tyr-L-Arg-O-alkyl^{B} avec acyl^{A} = une chaîne alkyle de C1 à C18, particulièrement préférée de C1 à C4 et alkyl^{B} = une chaîne alkyle de C1 à C18, particulièrement préférée de C8 à C16. Une réalisation particulièrement préférée est constituée par le peptide N-Acétyl-L-Tyr-L-Arg-O-hexadécyle. Il est obtenu par synthèse de la façon suivante :
On chauffe 300 ml de toluène à 100°C, on y ajoute 20 g d'hexadécanol et 34 g d'APTS (acide paratoluène sulfonique), puis graduellement on ajoute 17 g de L-Arginine.HCl. On estérifie à reflux pendant la nuit. On précipite au TEA, on filtre sur fritté et on sèche le précipité (ester hexadécyle de L-arginine). Ensuite, on prépare 5.6 g de N-acétyl-L-tyrosine dans 75 ml de THF, on y ajoute 3 g de N-hydroxysuccinimide dans 20 ml de THF. On rajoute une solution de 5.3 g DCC (Dicyclohexylcarbodiimide) dans 20 ml de dichlorométhane. Après formation quantitative de DCU et filtration, on ajoute la solution de l'ester de L-Arginine dans THF (14 g dans 200 ml de THF) et on laisse agir pendant 48 heures à température ambiante. On évapore le solvant et on cristallise à l'eau, puis on filtre et on sèche. Le produit obtenu (18 g) est caractérisé par chromatographie sur couche mince, HPLC, infrarouge et point de fusion (140-143°C).

Ces peptides ne sont généralement pas solubles dans l'eau, mais peuvent être dissous dans les solvants classiques cosmétiques ou dermopharmaceutiques comme l'éthanol, le propanol ou l'isopropanol, le propylène glycol, glycérine, le butylène glycol, l'ethoxydiglycol, le polyéthylène glycol), les éthers méthyliques ou éthyliques des diglycols, les polyols cycliques, les diglycols éthoxylés ou propoxylées ou tout mélange de ces solvants. L'homme de l'art connaît les méthodes de solubilisation préalable de ce genre de molécules, Les peptides peuvent également être préalablement incorporés dans des vecteurs cosmétiques comme les liposomes, les chylomicrons, les macro-, micro- et nanoparticules ainsi que les macro-, micro- et nanocapsules, ou être absorbés sur des polymères organiques poudreux, les talcs, bentonites et autres supports minéraux.

Ces solutions ou préparations peuvent ensuite être utilisées dans les crèmes, lotions, pommades et autres formes galéniques cosmétiques et dermopharmaceutiques.

A titre d'exemple, on cite une préparation d'un peptide représentatif de l'invention:

### Exemple n° 1:

| | |
|---|---|
| 1,3 Butylène glycol | 40% |
| Tween^{R} 20 | 1% |
| Ethoxydiglycol | 35% |
| Propylène glycol | 22% |
| N-Acétyl-L-Tyr-L-Arg-O-Hexadécyle | 2%. |

### Exemple n° 2:

| | |
|---|---|
| Eau | 20% |
| Ethanol | 30% |
| Isopropanol | 30% |
| Glycérine | 17% |
| N-Propyl-L-Tyr-L-Arg-O-Octadecyl | 3%. |

### Exemple n° 3:

Solution du peptide incorporée dans une crème de type apaisante:

| | |
|---|---|
| Brij^{R} 721 | 2.4 |
| Brij^{R} 72 | 2.6 |
| Arlamol^{R} E | 8.0 |
| Cire d'abeille | 0.5 |
| Abil^{R} ZP 2434 | 3.0 |
| Propylène glycol | 3.0 |
| Carbopol^{R} 941 | 0.25 |
| Triéthanolamine | 0.25 |
| solution du peptide de l'exemple n° 1 | 5.0 |
| Eau, conservateurs, parfums | qsp 100 g. |

Les effets cosmétiques ou dermopharmaceutiques des peptides objets du brevet ont été mis en évidence dans des tests *in vivo* :

### Exemple n° 4:

Vingt personnes volontaires ont été sélectionnées pour ce test. On applique sur chacune des personnes une sonde chauffante avec thermomètre intégré qui permet de chauffer une petite zone de la peau et d'en mesurer la température. Les personnes indiquent - à différents intervalles - les dépassements des seuils prédéfinis de "tiédeur", "chaleur", "très forte chaleur" et "douleur". Après avoir appris ces notions et leurs propres seuils de sensation, les personnes appliquent la crème contenant le peptide à tester sur la peau, sur le site de mesure. Après des temps d'attente variables (entre 15 minutes et 4 heures), les seuils de sensibilités à la chaleur sont de nouveau déterminés de la même façon. Il est ainsi possible de mesurer une éventuelle diminution de la sensibilité (effet apaisant, antalgique), en comparaison avec un placebo ne contenant pas de peptide.

On calcule un index d'effet apaisant par palier de chaleur.

L'efficacité des peptides objets du brevet est démontrée dans ce test: la diminution de la sensibilité à la chaleur est notable après 30 minutes et significative après 2 heures. L'indice d'effet apaisant augmente de 62% à 30 minutes, de 210% après 2 heures et de 80% après 4 heures.

Les peptides objets du présent brevet peuvent être utilisés dans toute forme galénique habituellement utilisée en formulation cosmétique ou dermopharmaceutique: émulsions H/E et E/H, laits, lotions, gels, pommades, baumes, mousses, huiles corporelles, lotions capillaires, shampooings, savons, sticks et crayons, sprays, sans que cette liste soit limitative.

La concentration d'utilisation de ces peptides dans le produit cosmétique fini peut varier entre 0.001 et 10% (p/p), préférentiellement entre 0.01 et 1% au poids de la composition totale.

Les peptides objets du présent brevet peuvent être combinés dans les compositions cosmétiques avec tout autre ingrédient habituellement utilisé en cosmétique: lipides d'extraction et/ou de synthèse, polymères gélifiants et viscosants, tensioactifs et émulsifiants, principes actifs hydro- ou liposolubles, extraits de plantes, extraits tissulaires, extraits marins.

Les peptides sous toutes leurs formes galéniques (poudre, solution, émulsion) peuvent être utilisés dans les domaines cosmétique et dermopharmaceutique pour leur activité calmante, antalgique. Ils sont avantageusement employés dans les produits pour peaux sensibles, les crèmes solaires et après-solaires, les baumes après-rasage, les crèmes dépilatoires ou après-épilation, sans que cette liste soit exhaustive. Leur utilisation est donc préconisée pour tous les soins de la peau ou du cuir chevelu, particulièrement l'atténuation des sensations d'irritation, de douleur bénigne, des effets de chaleur, de froid, du frottement ou des agressions mécaniques de la peau.

## Revendications

1. Peptides synthétiques à usage cosmétique ou dermopharmaceutique correspondant à la formule générale : R1-L-Tyr-L-Arg-R2 où R1= un groupe R3-C=O avec R3 = une chaîne alkyle de C1 à C20, linéaire ou ramifiée, saturée ou insaturée, hydroxylée ou non, ou avec R3 = un groupe aryle, aryle-alkyle ou alkyloxy, et où R2 = un groupe O-R4 avec R4= une chaîne alkyl de C1 à C20, ou R2 = un groupe NH2, NHX ou NXX avec X = une chaîne alkyle de C1 à C4.

2. Peptides synthétiques à usage cosmétique ou dermopharmaceutique selon la revendication 1 **caractérisés en ce que** les peptides sont obtenus soit par synthèse peptidique en milieu homogène ou hétérogène selon les méthodes classiques, soit par synthèse enzymatique en milieu préférentiellement anhydre.

3. Peptides synthétiques à usage cosmétique ou dermopharmaceutique selon la revendication 1 **caractérisés en ce que** les résidus R1= R3-C=O avec R3 = chaîne aliphatique de C1 à C18 et R2 =O-R4 avec R4 = chaîne linéaire saturée ou non de C1 à C18 représentent chacun une chaîne aliphatique linéaire saturée ou insaturée de 1 à 18 carbones.

4. Peptides synthétiques à usage cosmétique ou dermopharmaceutique selon la revendication 1 **caractérisés en ce que** le peptide est préférentiellement le N-Acétyl-L-Tyr-L-Arg-O-hexadécyle.

5. Compositions cosmétiques ou dermopharmaceutiques **caractérisées en ce qu'**elles contiennent au moins un peptide selon l'une quelconque des revendications de 1 à 4, préalablement solubilisés dans des solvants utilisables dans les domaines cosmétique et dermopharmaceutique comme l'eau, l'éthanol, le propanol ou l'isopropanol, le propylène glycol, le butylèneglycol, la glycérine, le polyéthylène glycol, l'ethoxydiglycol, les éthers méthyliques ou éthyliques des diglycols, les polyols cycliques, les diglycols éthoxylés ou propoxylées ou tout mélange de ces solvants.

6. Compositions cosmétiques ou dermopharmaceutiques **caractérisées en ce qu'**elles contiennent au moins un peptide selon l'une quelconque des revendications de 1 à 4 préalablement incorporé dans des vecteurs cosmétiques comme les liposomes, les chylomicrons, les macro-, micro- et nanoparticules ainsi que les macro-, micro- et nanocapsules, ou absorbé sur des polymères organiques poudreux, les talcs, bentonites et autres supports minéraux.

7. Compositions cosmétiques ou dermopharmaceutiques selon la revendication 5 ou 6 **caractérisées en ce qu'**elles contiennent le ou les peptides à des concentrations qui peuvent varier entre 0.001% (p/p) et 10%, préférentiellement entre 0.01 et 1% (p/p) en poids de la composition totale.

8. Compositions cosmétiques ou dermopharmaceutiques selon l'une quelconques des revendications de 5 à 7 **caractérisées en ce qu'**elles représentent toute forme galénique employée en cosmétique ou dermopharmacie à savoir les émulsions H/E et E/H, laits, lotions, gels, pommades, baumes, mousses, huiles corporelles, lotions capillaires, shampooings, savons, sticks et crayons, sprays.

9. Compositions cosmétiques ou dermopharmaceutiques selon l'une quelconques des revendications de 5 à 8 **caractérisées en ce que** les peptides selon l'une quelconque des revendications de 1 à 4 sont combinés dans les produits finis avec tout autre ingrédient habituellement utilisé en cosmétique ou dermopharmacie: lipides d'extraction et/ou de synthèse, polymères gélifiants et viscosants, tensioactifs, émulsifiants, principes actifs hydro- ou liposolubles, extraits de plantes, extraits tissulaires, extraits marins.

10. Utilisations des peptides selon l'une quelconque de revendications de 1 à 4 dans les compositions cosmétiques ou dermopharmaceutiques selon l'une quelconque des revendications de 5 à 9 pour les soins de la peau ou du cuir chevelu, particulièrement l'atténuation des sensations d'irritation, de douleur bénigne, des effets de chaleur, de froid, du frottement ou des agressions mécaniques de la peau.

## Claims

1. Synthetic peptides for cosmetic or dermopharmaceutical use corresponding to the general formula: R1-L-Tyr-L-Arg-R2, where R1 = an R3-C=O group with R3 = a C1 to C20 alkyl chain which is linear or branched, saturated or unsaturated, hydroxylated or non-hydroxylated, or with R3 = an aryl, aryl-alkyl or alkyloxy group, and where R2 = an O-R4 group with R4 = a C1 to C20 alkyl chain, or R2 = an NH2, NHX or NXX group with X = a C1 to C4 alkyl chain.

2. Synthetic peptides for cosmetic or dermopharmaceutical use according to claim 1, **characterised in that** the peptides are obtained either by peptide synthesis in a homogenous or heterogeneous medium according to standard methods or by enzymatical synthesis in a preferably anhydrous medium.

3. Synthetic peptides for cosmetic or dermopharmaceutical use according to claim 1, **characterised in that** the residues R1 = R3-C=O, with R3 = a C1 to C18 aliphatic chain and R2 = O-R4, with R4 = a saturated or non-saturated C1 to C18 chain, each represents an aliphatic, linear, saturated or non-saturated chain having 1 to 18 carbons.

4. Synthetic peptides for cosmetic or dermopharmaceutical use according to claim 1, **characterised in that** the peptide is preferably N-acetyl-L-Tyr-L-Arg-O-hexadecyl.

5. Cosmetic or dermopharmaceutical compositions, **characterised in that** they contain at least one peptide according to any of the claims from 1 to 4, first made soluble in solvents which can be used in the cosmetic or dermopharmaceutical spheres, such as water, ethanol, propanol or isopropanol, propylene glycol, butylene glycol, glycerine, polyethylene glycol, ethoxydiglycol, methylic or ethylic diglycol ethers, cyclic polyols, ethoxylated or propoxylated diglycols or any mixture of these solvents.

6. Cosmetic or dermopharmaceutical compositions, **characterised in that** they contain at least one peptide according to any of the claims from 1 to 4, first incorporated into cosmetic vehicles such as liposomes, chylomicrons, macro-, micro- and nanoparticles and also macro-, micro- and nanocapsules, or absorbed on powdery organic polymers, talcs, bentonites and other mineral carriers.

7. Cosmetic or dermopharmaceutical compositions according to claim 5 or 6, **characterised in that** they contain the peptide or peptides in concentrations which can vary between 0.001% (p/p) and 10%, preferably between 0.01 and 1% (p/p) by weight of the total composition.

8. Cosmetic or dermopharmaceutical compositions according to any of the claims from 5 to 7, **characterised in that** they represent any galenic form used in cosmetics or dermopharmaceuticals, i.e. H/E and E/H emulsions, milks, lotions, gels, ointments, foams, body oils, capillary lotions, shampoos, soaps, sticks and pencils, sprays.

9. Cosmetic or dermopharmaceutical compositions according to any of the claims from 5 to 8, **characterised in that** the peptides according to any of the claims from 1 to 4 are combined in the finished products with any other ingredient normally used in cosmetics or dermopharmaceuticals: extraction and/or synthesis lipids, gelling and viscosifying polymers, surfactants, emulsifiers, hydro- or liposoluble active principles, plant extracts, tissue extracts, marine extracts.

10. Uses of peptides according to any of the claims from 1 to 4 in cosmetic or dermopharmaceutical compositions according to any of the claims from 5 to 9 for care of the skin or the scalp, particularly the easing of feelings of irritation, of mild pain, of the effects of heat, of cold, of friction or of mechanical effects damaging to the skin.

## Patentansprüche

1. Synthetische Peptide zur kosmetischen oder hautpharmakologischen Verwendung entsprechend der allgemeinen Formel: R1-L-Tyr-L-Arg-R2, wobei R1 = eine Gruppe R3-C=O ist mit R3 = eine lineare oder verzweigte, gesättigte oder ungesättigte, hydroxylierte oder nicht hydroxylierte Alkylkette mit C1 bis C20, oder mit R3 = eine Aryl-, Aryl-Alkyl- oder Alkyloxy-Kette, und wobei R2 = eine Gruppe O-R4 ist mit R4 = eine Alkylkette mit C1 bis C20 oder R2 = eine Gruppe NH2, NHX oder NXX mit X = eine Alkylkette mit C1 bis C4.

2. Synthetische Peptide zur kosmetischen oder hautpharmakologischen Verwendung entsprechend Anspruch 1, **dadurch gekennzeichnet, dass** die Peptide entweder durch peptidische Synthese in einem homogenen oder heterogenen Medium entsprechend den herkömmlichen Methoden oder durch enzymatische Synthese in einem vorzugsweise anhydriden Medium erhalten sind.

3. Synthetische Peptide zur kosmetischen oder hautpharmakologischen Verwendung entsprechend Anspruch 1, **dadurch gekennzeichnet, dass** die Reste R1 = R3-C=O mit R3 = eine aliphatische Kette mit C1 bis C18 und R2 = O-R4 mit R4 = eine lineare gesättigte Kette oder eine Kette nicht mit C1 bis C18, jeweils eine gesättigte oder ungesättigte, lineare, aliphatische Kette mit 1 bis 18 Kohlenstoffen darstellen.

4. Synthetische Peptide zur kosmetischen oder hautpharmakologischen Verwendung entsprechend Anspruch 1, **dadurch gekennzeichnet, dass** das Peptid vorzugsweise vom Typ N-Acetyl-L-Tyr-Arg-O-Hexadecyl ist.

5. Kosmetische oder hautpharmakologische Zusammensetzungen, **dadurch gekennzeichnet, dass** sie mindestens ein Peptid entsprechend einem der Ansprüche 1 bis 4 enthalten, das vorab löslich gemacht wurde in für den kosmetischen und hautpharmakologischen Bereich vorgesehenen Lösungsmitteln wie Wasser, Ethanol, Propanol oder Isopropanol, Glykol-Propylen, Butylen-Glykol, Glyzerin, Polyethylen-Glykol, Ethoxy-Diglykol, Ethyl- oder Methyl-Ether von Diglykolen, zyklische Polyole, ethoxylische oder propoxylische Diglykole oder jede beliebige Mischung dieser Lösungsmittel.

6. Kosmetische oder hautpharmakologische Zusammensetzungen, **dadurch gekennzeichnet, dass** sie mindestens ein Peptid entsprechend einem der Ansprüche 1 bis 4 enthalten, das vorab in kosmetische Vektoren eingebracht wurde wie Liposome, Chylomikrone, Makro-, Mikro und Nanopartikel sowie Makro, Mikro- und Nanokapseln oder auf pulvrigen, organischen Polymeren, Talken, Bentonit oder anderen mineralischen Medien absorbiert wurde.

7. Kosmetische oder hautpharmakologische Zusammensetzungen gemäß einem der Ansprüche 5 bis 6, **dadurch gekennzeichnet, dass** sie das Peptid oder die Peptide in Konzentrationen enthalten, die zwischen 0,001 % (p/p) und 10 %, vorzugsweise zwischen 0,01 und 1 % (p/p), bezogen auf das Gewicht der Gesamtzusammensetzung, variieren können.

8. Kosmetische oder hautpharmakologische Zusammensetzungen entsprechend einem der Ansprüche 5 bis 7, **dadurch gekennzeichnet, dass** sie in beliebiger galenischer Form formuliert sind, die in der Kosmetik- oder Hautpharmakologie verwendet wird, nämlich WO-Emulsionen und OW-Emulsionen, Milch, Lotionen, Gele, Pomaden, Balsame, Schäume, Körperöle, Haarlotionen, Shampoos, Seifen, Stifte und Schminkstifte, Sprays.

9. Kosmetische oder hautpharmakologische Zusammensetzungen entsprechend einem der Ansprüche 5 bis 8, **dadurch gekennzeichnet, dass** die Peptide entsprechend einem der Ansprüche 1 bis 4 in den Endprodukten kombiniert werden können mit jeder anderen Ingredienz, die üblicherweise im Kosmetik- oder Dermopharmakologiebereich verwendet wird: extrahierte und/oder synthetische Lipide, gelbildende und zähflüssigmachende Polymere, Tenside, Emulgatoren, wasser- oder fettlösliche Wirkstoffe, Pflanzenextrakte, Gewebeextrakte, Meeresextrakte.

10. Verwendung der Peptide entsprechend einem der Ansprüche 1 bis 4 in den kosmetischen oder hautpharmakologischen Zusammensetzungen entsprechend einem der Ansprüche 5 bis 9 zur Haut- oder Kopfhautpflege, insbesondere zur Milderung von Irritationsempfindungen, gutartigen Schmerzen, Auswirkungen von Wärme, Kälte, Reibung oder mechanischen Angriffen auf die Haut.
